# EUROPEAN PATENT SPECIFICATION

(11) **EP 3 833 418 B1**
(45) Date of publication and mention of the grant of the patent: **07.10.2026**
(21) Application number: 19846257.4
(22) Date of filing: 08.08.2019
(51) Int. Cl.: A61K 33/00, A61P 11/00, A61P 31/04, A61P 43/00, A61B 5/0205, A61B 5/145, A61B 5/1455, A61B 5/08, A61M 15/00, A61M 15/08, A61M 16/00, A61M 16/06, A61M 16/10, A61M 16/12

(54) **IMPROVED DOSAGE REGIMEN FOR THE ADMINISTRATION OF INHALED NITRIC OXIDE (INO) IN THE TREATMENT OF HYPOXEMIA**
VERBESSERTES DOSIERUNGSSCHEMA FÜR DIE VERABREICHUNG VON INHALIERTEM STICKSTOFFMONOXID (INO) ZUR BEHANDLUNG VON HYPOXÄMIE
AMÉLIORATION DU SCHÉMA POSOLOGIQUE POUR L'ADMINISTRATION D'OXYDE NITRIQUE INHALÉ (INO) DANS LE TRAITEMENT DE L'HYPOXÉMIE

(30) Priority: 10.08.2018 US 201862717433 P
(43) Date of publication of application: 16.06.2021
(73) Proprietor: Mallinckrodt Pharmaceuticals Ireland Limited, Dublin 15 (IE)
(72) Inventor: SHAH, Parag, Morristown, NJ 07960 (US); QUINN, Deborah, Morristown, NJ 07960 (US)
(74) Representative: Boult Wade Tennant LLP
(86) International application number: PCT/US2019/045806
(87) International publication number: WO 2020/033768

(56) References cited:
- WO-A1-2019/133776
- US-A- 5 890 490
- US-A1- 2006 201 504
- US-A1- 2014 275 901
- US-A1- 2017 165 294
- FARBER H.W. ET AL: "5018 A phase 3 study in an enriched population of pulmonary arterial hypertension patients with long term oxygen use demonstrating improved exercise tolerance with use of ambulatory inhaled nitric oxide", EUROPEAN HEART JOURNAL, vol. 38, no. suppl_1, 1 August 2017 (2017-08-01), GB, pages 1051 - 1051, XP055826732, ISSN: 0195-668X, DOI: 10.1093/eurheartj/ehx493.5018
- RAYMOND L BENZA ET AL: "A Phase 2 Placebo-Controlled, Randomized, Double-Blind Clinical Study to Assess the Efficacy, Safety and Tolerability of Two Doses of Pulsed, Inhaled Nitric Oxide (iNO) in Patients with WHO Group 1 Pulmonary Arterial Hypertension (PAH): 12 Month Interim Analysis of Open Label Extension", vol. 192, 1 January 2016 (2016-01-01), pages A7322, XP009534464, ISSN: 1073-449X, Retrieved from the Internet <URL:https://www.atsjournals.org/doi/abs/10.1164/ajrccm-conference.2016.193.1_MeetingAbstracts.A7322> [retrieved on 20220328]
- MICHAEL JOHN?R. ET AL: "Inhaled Nitric Oxide Versus Conventional Therapy : Effect on Oxygenation in ARDS", AMERICAN JOURNAL OF RESPIRATORY AND CRITICAL CARE MEDICINE, vol. 157, no. 5, 1 May 1998 (1998-05-01), US, pages 1372 - 1380, XP055904384, ISSN: 1073-449X, DOI: 10.1164/ajrccm.157.5.96-10089
- TEMAN N. R. ET AL: "Inhaled Nitric Oxide to Improve Oxygenation for Safe Critical Care Transport of Adults With Severe Hypoxemia", AMERICAN JOURNAL OF CRITICAL CARE, vol. 24, no. 2, 1 March 2015 (2015-03-01), US, pages 110 - 117, XP055904376, ISSN: 1062-3264, DOI: 10.4037/ajcc2015570
- ANON.: "Fraction of Inspired Oxygen (FiO2)", 28 March 2022 (2022-03-28), XP055905827, Retrieved from the Internet <URL:https://louisville.edu/medicine/departments/medicine/divisions/gimedicine/physician-resources/calculators-and-tools-files/fraction-of-inspired-oxygen-pdf/view> [retrieved on 20220328]

## Description

### FIELD OF THE INVENTION

The present application relates generally to inhaled nitric oxide (iNO) for therapeutic improvement to severe hypoxemia.

### BACKGROUND OF THE INVENTION

Nitric oxide (NO) is a gas that, when inhaled, acts to dilate blood vessels in the lungs, improving oxygenation of the blood and reducing pulmonary hypertension. Because of this, nitric oxide is provided as a therapeutic gas in the inspiratory breathing phase for patients having difficulty breathing due to a disease state, for example, pulmonary arterial hypertension (PAH), chronic obstructive pulmonary disorder (COPD), combined pulmonary fibrosis and emphysema (CPFE), cystic fibrosis (CF), idiopathic pulmonary fibrosis (IPF), emphysema, interstitial lung disease (ILD), chronic thromboembolic pulmonary hypertension (CTEPH), chronic high altitude sickness, or other lung disease.

While NO may be therapeutically effective when administered under the appropriate conditions, it can also become toxic if not administered correctly. NO reacts with oxygen to form nitrogen dioxide (NO₂), and NO₂ can be formed when oxygen or air is present in the NO delivery conduit. NO₂ is a toxic gas which may cause numerous side effects, and the Occupational Safety & Health Administration (OSHA) provides that the permissible exposure limit for general industry is only 5 ppm. Thus, it is desirable to limit exposure to NO₂ during NO therapy.

Effective dosing of NO is based on a number of different variables, including quantity of drug and the timing of delivery. Several patents have been granted relating to NO delivery, including US Patent Nos. 7,523,752; 8,757,148; 8,770,199; and 8,803,717, and a Design Patent D701,963 for a design of an NO delivery device. Additionally, there are pending applications relating to delivery of NO, including US2013/0239963 and US2016/0106949.

The maximum level of oxygen delivery on an outpatient basis is 10 L/min. Some patients with severe hypoxemia due to, for example, advanced lung disease, may have an oxygenation level that drops to less than 88% while already on the maximum 10 L/min oxygen. These patients are unable to be discharged from the hospital because oxygen saturation less than 88% can lead to life threatening situations. In the hospital, these patients are maintained on both nasal cannula oxygen and a high flow oxygen mask. The need to maintain high levels of oxygen therapy prevents these patients from being discharged from the hospital.

Inhaled NO is an effective vasodilator, for example, for use in pediatric pulmonary hypertension. Using continuous flow devices for delivery of iNO, it has been shown that both in adults and newborn infants with severe hypoxemia in the acute care setting, iNO improves oxygenation (Teman NR, et al, AJCC Journal, 2018; Tang SF, et al, Arch Dis Child, 1998). However, continuous flow iNO requires large tanks of gas and adequate ventilation to prevent buildup of environmental NO and other byproducts. This limits the use of continuous iNO to hospital settings and prevents the ability to treat patients in the home or in an ambulatory setting. There remains a need for patients requiring improved oxygenation to be able to receive high levels of oxygen through use of iNO delivery devices outside of a hospital setting, that is, in an outpatient, ambulatory, or home environment so that the patient is not required to be hospitalized to receive the appropriate oxygen therapy.

Farber et al (2019) Eur. Heart J. 38, 1051 reports on an ongoing phase 3 study assessing the impact of inhaled nitric oxide on exercise tolerance in a population of pulmonary arterial hypertension patients.

Denza et al (2016) Am. J. Resp. Crit. Care 192, A7322 reports on a phase 2 study assessing the efficacy, safety and tolerability of inhaled nitric oxide in patients with WHO Group 1 Pulmonary Arterial Hypertension (PAH).

### SUMMARY OF THE INVENTION

The present invention provides inhaled nitric oxide (iNO) for use in a method of improving oxygen saturation in a patient having an initial oxygen saturation of less than 88% when receiving continuous oxygen therapy at a flow of 10L/min., said method comprising:
(a) administering said iNO to said patient until the oxygen saturation is at least 88%, wherein said iNO is delivered in a continuous manner or in a pulsatile manner; and
(b) continuing said oxygen administration together with iNO administration, wherein said iNO is delivered in a pulsatile manner during a portion of the inspiratory phase of a breath, wherein:
   (i) delivery of at least fifty percent of the dose of iNO occurs within the first third of the total inspiratory phase of a breath;
   (ii) delivery of the dose of iNO occurs within the first two-thirds of the total inspiratory phase of a breath;
   (iii) delivery of at least ninety percent of the dose of iNO occurs within the first two-thirds of the total inspiratory phase of a breath; or
   (iv) delivery of at least seventy percent of the dose of iNO occurs within the first half of the total inspiratory phase of a breath.

In some embodiments, in step (b), delivery of the dose of iNO occurs within the first third of the inspiratory phase of a breath. In some embodiments, in step (b), the iNO is delivered in a series of pulses. In some embodiments, in step (b), the iNO is delivered at 75 mcg/kg/hr or 25 mcg/kg/hr.

In some embodiments, the patient has idiopathic pulmonary fibrosis (IPF), pulmonary arterial hypertension (PAH), pulmonary hypertension (PH), chronic obstructive pulmonary disorder (COPD), cystic fibrosis (CF), interstitial lung disease (ILD), combined pulmonary fibrosis and emphysema (CPFE), chronic high altitude sickness, chronic thromboembolic pulmonary hypertension (CTEPH), emphysema, or pneumonia.

Described herein is a method for improving oxygen saturation in a patient suffering from hypoxemia. The method comprises delivering a dose of iNO in a pulsatile manner. In an example , the iNO is delivered during a portion of the inspiratory phase of a breath. In an example, the patient is receiving continuous oxygen therapy at a flow of about 10L/min.

The invention provides inhaled nitric oxide (iNO) for use in a method of improving oxygen saturation in a patient having an initial oxygen saturation of less than 88% when receiving continuous oxygen therapy at a flow of 10L/min. according to the claims. In some embodiments, the method comprises administering iNO to the patient in an inpatient setting until the oxygen saturation is at least 88%, and continuing said iNO and oxygen administration in an outpatient setting. In an embodiment of the invention, the iNO delivered in an outpatient setting is delivered in a pulsatile manner.

Described herein is a method for reducing costs associated with patient hospitalization is described. The method comprises identifying a patient with an oxygen saturation level of below 88% while on continuous oxygen therapy at 10L/min., delivering iNO to the patient until the oxygen saturation level rises to above 88%, discharging the patient from the hospital and continuing to deliver iNO in a pulsatile manner to the patient together with continuous oxygen therapy in an outpatient setting.

Described herein is a method for improving quality of life for a hospitalized patient is described. The method comprises identifying a patient with an oxygen saturation level of below 88% while on continuous oxygen therapy at 10L/min., delivering iNO to the patient until the oxygen saturation level rises to above 88%, discharging the patient from the hospital and continuing to deliver iNO in a pulsatile manner to the patient together with continuous oxygen therapy in an outpatient setting.

Described herein is a method for reducing patient hospitalization time is described. The method comprises identifying a patient with an oxygen saturation level of below 88% while on continuous oxygen therapy at 10L/min., delivering iNO to the patient until the oxygen saturation level rises to above 88%, discharging the patient from the hospital and continuing to deliver iNO in a pulsatile manner to the patient together with continuous oxygen therapy in an outpatient setting.

Various embodiments are listed above and will be described in more detail below.

### BRIEF DESCRIPTION OF THE DRAWINGS

The foregoing summary, as well as the following detailed description of the invention, will be better understood when read in conjunction with the appended drawings.

So that the manner in which the above recited features of the present invention can be understood in detail, a more particular description of the invention, briefly summarized above, may be had by reference to embodiments, some of which are illustrated in the appended drawings. It is to be noted, however, that the appended drawings illustrate only typical embodiments of this invention and are therefore not to be considered limiting of its scope, for the invention may admit to other equally effective embodiments.
FIG. 1 is a graph demonstrating a single measurement of a breath.
FIG. 2 is a graph demonstrating measurement of a delivered pulse of nitric oxide to a patient according to the present invention.

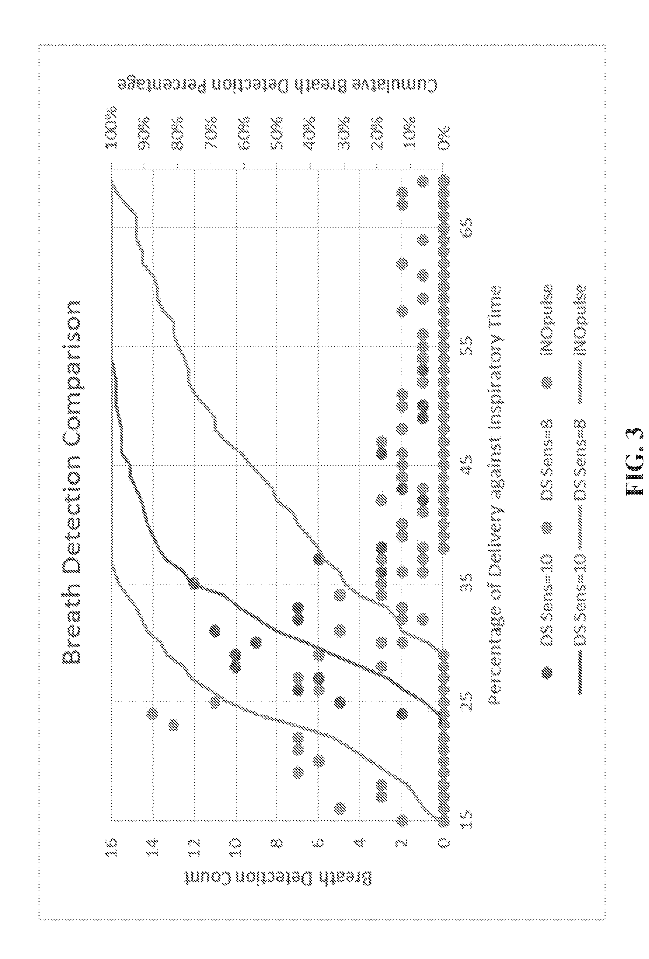

FIG. 3 is a graph demonstrating detection of breaths as a percentage of nitric oxide delivery over total inspiratory time. The orange line represents a breath sensitivity setting of 8 of 10 (e.g., 80% of maximum sensitivity) on Device 1, the blue line represents a breath sensitivity setting of 10 of 10 (e.g., maximum sensitivity) on Device 1, and the green line represents a fixed breath sensitivity setting of 10 on Device 2. The green line demonstrates that about 93% of the nitric oxide dose is delivered during the first 33% (or first third) of total inspiratory time, and 100% of the nitric oxide dose is delivered during the first 50% (or first half) of total inspiratory time. The blue line demonstrates that about 62% of the nitric oxide dose is delivered during the first 33% (or first third) of total inspiratory time, about 98% is delivered during the first 50% (or first half) of total inspiratory time, and 100% is delivered during the first 67% (or first two-thirds) of total inspiratory time. The orange line demonstrates that about 17% of the nitric oxide dose is delivered during the first 33% (or first third) of total inspiratory time, about 72% is delivered during the first 50% (or first half) of total inspiratory time, and about 95% during the first 67% (or first two-thirds) of total inspiratory time.
FIG. 4 depicts the combined results described in FIG. 3.

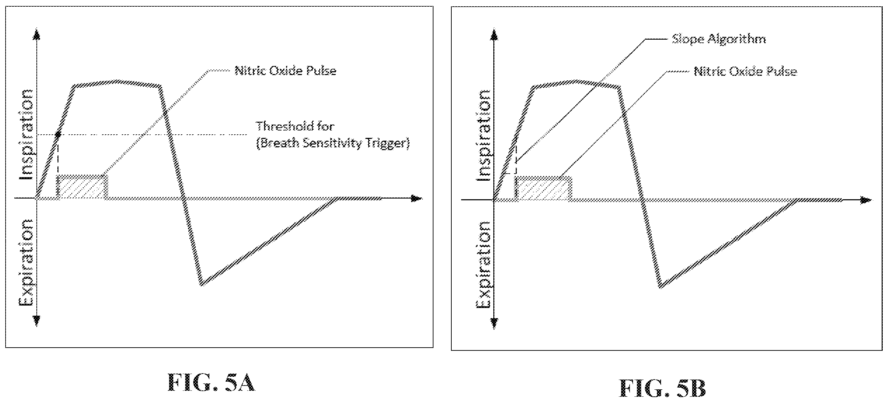

FIGS. 5A and 5B depict an algorithm for breath detection and delivery of nitric oxide. FIG. 5A demonstrates a threshold algorithm. FIG. 5B demonstrates a slope algorithm.

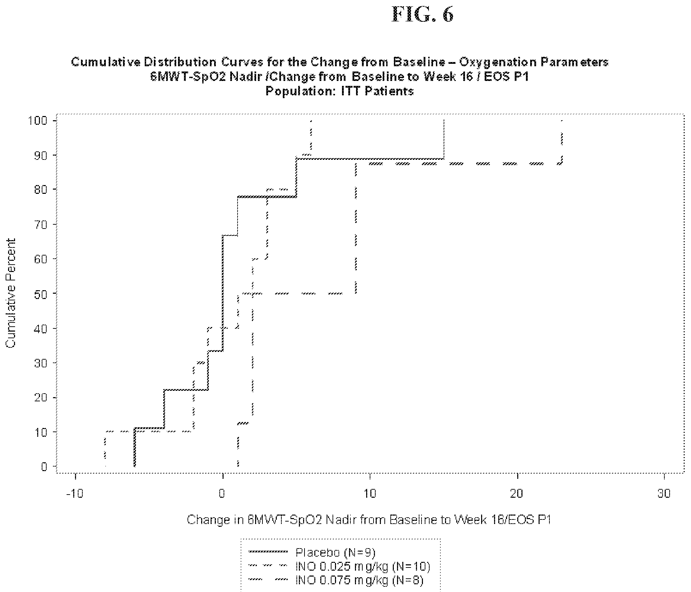

FIG. 6 is a graph demonstrating cumulative distribution curves for the change in SpO₂ Nadir during the 6MWT for subjects on placebo, iNO at 25mg/kg, and iNO at 75mg/kg.

### DETAILED DESCRIPTION OF THE INVENTION

Unless defined otherwise, all technical and scientific terms used herein have the same meaning as is commonly understood by one of skill in the art to which this invention belongs.

Before describing several exemplary embodiments of the invention, it is to be understood that the invention is not limited to the details of construction or process steps set forth in the following description. The invention is capable of other embodiments and of being practiced or being carried out in various ways.

Reference throughout this specification to "one embodiment," "certain embodiments," "one or more embodiments" or "an embodiment" means that a particular feature, structure, material, or characteristic described in connection with the embodiment is included in at least one embodiment of the invention. Thus, the appearances of the phrases such as "in one or more embodiments," "in certain embodiments," "in one embodiment" or "in an embodiment" in various places throughout this specification are not necessarily referring to the same embodiment of the invention. Furthermore, the particular features, structures, materials, or characteristics may be combined in any suitable manner in one or more embodiments.

Although the invention herein has been described with reference to particular embodiments, it is to be understood that these embodiments are merely illustrative of the principles and applications of the present invention.

### Definitions

The term "effective amount" or "therapeutically effective amount" refers to that amount of a compound or combination of compounds as described herein that is sufficient to effect the intended application including, but not limited to, disease treatment. A therapeutically effective amount may vary depending upon the intended application (*in vitro* or *in vivo*), or the subject and disease condition being treated (*e.g*., the weight, age and gender of the subject), the severity of the disease condition, the manner of administration, *etc.* which can readily be determined by one of ordinary skill in the art. The term also applies to a dose that will induce a particular response in target cells (*e.g*., the reduction of platelet adhesion and/or cell migration). The specific dose will vary depending on the particular compounds chosen, the dosing regimen to be followed, whether the compound is administered in combination with other compounds, timing of administration, the tissue to which it is administered, and the physical delivery system in which the compound is carried.

A "therapeutic effect" as that term is used herein, encompasses a therapeutic benefit and/or a prophylactic benefit. A prophylactic effect includes delaying or eliminating the appearance of a disease or condition, delaying or eliminating the onset of symptoms of a disease or condition, slowing, halting, or reversing the progression of a disease or condition, or any combination thereof.

When ranges are used herein to describe for example, dosing ranges, amounts of a component of a formulation, etc., all combinations and subcombinations of ranges and specific embodiments therein are intended to be included. Use of the term "about" when referring to a number or a numerical range means that the number or numerical range referred to is an approximation within experimental variability (or within statistical experimental error), and thus the number or numerical range may vary. The variation is typically from 0% to 15%, preferably from 0% to 10%, more preferably from 0% to 5% of the stated number or numerical range. The term "comprising" (and related terms such as "comprise" or "comprises" or "having" or "including") includes, for example, an example of any composition of matter, method or process that "consist of" or "consist essentially of" the described features.

For the avoidance of doubt, it is intended herein that particular features (for example integers, characteristics, values, uses, diseases, formulae, compounds or groups) described in conjunction with a particular aspect, embodiment or example herein are to be understood as applicable to any other aspect, embodiment or example described herein unless incompatible therewith. Thus such features may be used where appropriate in conjunction with any of the definition, claims or embodiments defined herein. All of the features disclosed in this specification (including any accompanying claims, abstract and drawings), and/or all of the steps of any method or process so disclosed, may be combined in any combination, except combinations where at least some of the features and/or steps are mutually exclusive. The invention is not restricted to any details of any disclosed embodiments.

In certain examples, a dose of a gas (e.g., NO) is administered in a pulse to a patient during an inspiration by the patient. It has been surprisingly discovered that nitric oxide delivery can be precisely and accurately delivered within the first two-thirds of total breath inspiration time and the patient obtains benefits from such delivery. Such delivery minimizes loss of drug product and risk of detrimental side effects increases the efficacy of a pulse dose which in turn results in a lower overall amount of NO that needs to be administered to the patient in order to be effective. Such delivery is useful for the treatment of various diseases, such as but not limited to idiopathic pulmonary fibrosis (IPF), pulmonary arterial hypertension (PAH), including Groups I-V pulmonary hypertension (PH), chronic obstructive pulmonary disorder (COPD), cystic fibrosis (CF), interstitial lung disease (ILD), combined pulmonary fibrosis and emphysema (CPFE), chronic high altitude sickness, chronic thromboembolic pulmonary hypertension (CTEPH), and emphysema, and is also useful as an antimicrobial, for example, in treating pneumonia.

Such precision may have further advantages in that only portions of the poorly ventilated lung area is exposed to NO. Hypoxia and issues with hemoglobin may also be reduced with such pulsed delivery, while NO₂ exposure is also more limited.

### Improving Oxygen Saturation

When a patient suffers from extreme hypoxemia (e.g., an SpO₂ level of lower than 88%) while on a maximum flow of continuous oxygen (e.g., 10L/min), the patient is required to be admitted to and/or stay in the hospital because hypoxemia at that level may cause life threatening injury and/or further illness. The need to maintain high levels of oxygen therapy prevents these patients from being discharged from the acute care setting (e.g., a hospital). Use of continuous flow iNO has been shown to improve oxygen saturation in patients with severe hypoxemia in an acute care setting. However, continuous flow iNO also requires hospitalization due to ventilation requirements and gas tank sizes. Thus, even if the patient's SpO₂ level rises to above the 88% threshold, the patient would still require hospitalization to receive the continuous iNO therapy.

Described herein is a method of improving oxygen saturation using pulsed dose delivery of iNO in order to reduce the hospitalization time and costs. The pulsed dose delivery method uses a portable, personal device, and delivers small, pulsed doses of iNO at specific times during inspiration, as described in more detail below, which obviates the need for large tanks, appropriate ventilation, and hospitalization. This gives the patient more freedom and comfort to get on with their life, and reduces hospitalization time and costs for the patient and for the healthcare system.

Described herein is a method for improving oxygen saturation in patients displaying little or no improvement to SpO₂ with long term oxygen therapy (LTOT). Patients must first have their SpO₂ levels reach or exceed a threshold to be discharged from the hospital. Thus, in an embodiment of the invention, pulsed iNO delivery may occur in the hospital setting. In another embodiment, when the threshold SpO₂ level is reached or exceeded, the patient may be discharged from the hospital setting and use or continue to use the pulsed iNO delivery system in an outpatient setting. In certain embodiments, the threshold SpO₂ level is between 80% and 90%, is between 82% and 88%, is between 84% and 86%. In certain embodiments, the threshold SpO₂ level is 80%, 81%, 82%, 83%, 84%, 85%, 86%, 87%, 88%, 89%, or 90%. In certain embodiments, the threshold SpO₂ level is 88%.

Described herein is a method for reducing hospitalization costs. The method includes identifying a patient with an oxygen saturation level of below 88% while on continuous oxygen therapy at 10L/min; delivering iNO (e.g., in a pulsatile manner as described herein or in a continuous manner) to said patient until said oxygen saturation level rises to above 88%; discharging said patient from the hospital; and continuing to deliver iNO in a pulsed manner to said patient together with continuous oxygen therapy in an outpatient setting. In certain embodiments, the oxygen saturation is maintained at or above 88% for the patient in the outpatient setting.

### A Device For Use With The Methods Described Herein

IDescribed herein is a device, e.g. a programmable device for delivering a dose of a gas (e.g., nitric oxide) to a patient in need. The device can include a delivery portion, a drug cartridge including a compressed gas for delivery to a patient, a breath sensitivity portion to detect a breath pattern in patient comprising a breath sensitivity setting, at least one breath detection algorithm for determining when to administer the compressed gas to the patient and a portion for administering the dose of nitric oxide to the patient through a series of one or more pulses.

In certain examples, the drug cartridge is replaceable.

In certain examples, the delivery portion includes one or more of a nasal cannula, a face mask, an atomizer, and a nasal inhaler. In certain examples, the delivery portion can further include a second delivery portion to permit the simultaneous administration of one or more other gases (e.g., oxygen) to a patient.

In certain examples, and as detailed elsewhere herein, the device includes an algorithm wherein the algorithm uses one or both of a threshold sensitivity and a slope algorithm, wherein the slope algorithm detects a breath when the rate of pressure drop reaches a predetermined threshold.

In an example , mechanically, a pulse dose of a gas can reduce, if not eliminate, venturi effects which would normally create problems for other gas sensors. For example, in the absence of the pulse doses, O₂ back pressure sensors may override delivery of O₂ when O₂ is administered simultaneously with another gas such as NO.

### Breath Patterns, Detection and Triggers

Breath patterns vary based on the individual, time of day, level of activity, and other variables; thus it is difficult to predetermine a breath pattern of an individual. A delivery system that delivers therapeutics to a patient based on breath pattern, then, should be able to handle a range of potential breath patterns in order to be effective.

In certain embodiments, the patient or individual can be any age, however, in more certain embodiments the patient is sixteen years of age or older.

In an example, the breath pattern includes a measurement of total inspiratory time, which as used herein is determined for a single breath. However, depending on context "total inspiratory time" can also refer to a summation of all inspiratory times for all detected breaths during a therapy. Total inspiratory time may be observed or calculated. In another embodiment, total inspiratory time is a validated time based on simulated breath patterns.

In an example, breath detection includes at least one and in some examples at least two separate triggers functioning together, namely a breath level trigger and/or a breath slope trigger.

In an example, a breath level trigger algorithm is used for breath detection. The breath level trigger detects a breath when a threshold level of pressure (e.g., a threshold negative pressure) is reached upon inspiration.

In an example, a breath slope trigger detects breath when the slope of a pressure waveform indicates inspiration. The breath slope trigger is, in certain instances, more accurate than a threshold trigger, particularly when used for detecting short, shallow breaths.

In an example, a combination of these two triggers provides overall a more accurate breath detection system, particularly when multiple therapeutic gases are being administered to a patient simultaneously.

In an example, the breath sensitivity control for detection of either breath level and/or breath slope is fixed. In an example, the breath sensitivity control for detection of either breath level or breath slope is adjustable or programmable. In an example, the breath sensitivity control for either breath level and/or breath slope is adjustable from a range of least sensitive to most sensitive, whereby the most sensitive setting is more sensitive at detecting breaths than the least sensitive setting.

In certain examples where at least two triggers are used, the sensitivity of each trigger is set at different relative levels. In one example where at least two triggers are used, one trigger is set a maximum sensitivity and another trigger is set at less than maximum sensitivity. In one example where at least two triggers are used and where one trigger is a breath level trigger, the breath level trigger is set at maximum sensitivity.

Oftentimes, not every inhalation/inspiration of a patient is detected to then be classified as an inhalation/inspiration event for the administration of a pulse of gas (e.g., NO). Errors in detection can occur, particularly when multiple gases are being administered to a patient simultaneously, e.g., NO and oxygen combination therapies.

Examples of the disclosure, and in particular an example which incorporates a breath slope trigger alone or in combination with another trigger, can maximize the correct detection of inspiration events to thereby maximize the effectiveness and efficiency of a therapy while also minimizing waste due to misidentification or errors in timing.

In certain example, greater than 50% of the total number of inspirations of a patient over a timeframe for gas delivery to the patient are detected. In certain example, greater than 75% of the total number of inspirations of a patient are detected. In certain example, greater than 90% of the total number of inspirations of a patient are detected. In certain example, greater than 95% of the total number of inspirations of a patient are detected. In certain example, greater than 98% of the total number of inspirations of a patient are detected. In certain example, greater than 99% of the total number of inspirations of a patient are detected. In certain example, 75% to 100% of the total number of inspirations of a patient are detected.

### Dosages and Dosing Regimens

In an embodiment of the invention, nitric oxide delivered to a patient is formulated at concentrations of about 3 to about 18mg NO per liter, about 6 to about 10 mg per liter, about 3 mg NO per liter, about 6 mg NO per liter, or about 18 mg NO per liter. The NO may be administered alone or in combination with an alternative gas therapy. In certain embodiments, oxygen (e.g., concentrated oxygen) can be administered to a patient in combination with NO.

In an embodiment of the present invention, a volume of nitric oxide is administered (e.g., in a single pulse) in an amount of from about 0.350mL to about 7.5mL per breath. In some embodiments, the volume of nitric oxide in each pulse dose may be identical during the course of a single session. In some embodiments, the volume of nitric oxide in some pulse doses may be different during a single timeframe for gas delivery to a patient. In some embodiments, the volume of nitric oxide in each pulse dose may be adjusted during the course of a single timeframe for gas delivery to a patient as breath patterns are monitored. In an embodiment of the invention, the quantity of nitric oxide (in ng) delivered to a patient for purposes of treating or alleviating symptoms of a pulmonary disease on a per pulse basis (the "pulse dose") is calculated as follows and rounded to the nearest nanogram value: 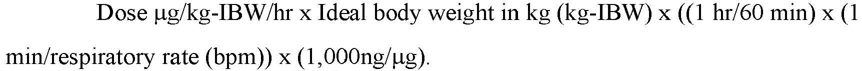 Dose µg/kg-IBW/hr x Ideal body weight in kg (kg-IBW) x ((1 hr/60 min) x (1 min/respiratory rate (bpm)) x (1,000ng/µg).

As an example, Patient A at a dose of 100 µg/kg IBW/hr has an ideal body weight of 75kg, has a respiratory rate of 20 breaths per minute (or 1200 breaths per hour): $100 μg / kg - IBW / hr \times 75 kg \times \left(1 hr/1200 breaths\right) X \left(1,000 ng/μg\right) = 6250 ng per pulse$

In certain embodiments, the 60/respiratory rate (ms) variable may also be referred to as the Dose Event Time. In another embodiment of the invention, a Dose Event Time is 1 second, 2 seconds, 3 seconds, 4 seconds, 5 seconds, 6 seconds, 7 seconds, 8 seconds, 9 seconds, or 10 seconds.

In an embodiment of the invention, a single pulse dose provides a therapeutic effect (e.g., a therapeutically effective amount of NO) to the patient. In another embodiment of the invention, an aggregate of two or more pulse doses provides a therapeutic effect (e.g., a therapeutically effective amount of NO) to the patient.

In an embodiment of the invention, at least about 300, about 310, about 320, about 330, about 340, about 350, about 360, about 370, about 380, about 390, about 400, about 410, about 420, about 430, about 440, about 450, about 460, about 470, about 480, about 490, about 500, about 510, about 520, about 530, about 540, about 550, about 560, about 570, about 580, about 590, about 600, about 625, about 650, about 675, about 700, about 750, about 800, about 850, about 900, about 950, or about 1000 pulses of nitric oxide is administered to a patient every hour.

In an embodiment of the invention, a nitric oxide therapy session occurs over a timeframe. In one embodiment, the timeframe is at least about 1 hour, about 2 hours, about 3 hours, about 4 hours, about 5 hours, about 6 hours, about 7 hours, about 8 hours, about 9 hours, about 10, hours, about 11 hours, about 12 hours, about 13 hours, about 14 hours, about 14 hours, about 15 hours, about 16 hours, about 17 hours, about 18 hours, or about 24 hours per day.

In an embodiment of the invention, a nitric oxide treatment is administered for a timeframe of a minimum course of treatment. In an embodiment of the invention, the minimum course of treatment is about 10 minutes, about 15 minutes, about 20 minutes, about 30 minutes, about 40 minutes, about 50 minutes, about 60 minutes, about 70 minutes, about 80 minutes, or about 90 minutes. In an embodiment of the invention, the minimum course of treatment is about 1 hour, about 2 hours, about 3 hours, about 4 hours, about 5 hours, about 6 hours, about 7 hours, about 8 hours, about 9 hours, about 10, hours, about 11 hours, about 12 hours, about 13 hours, about 14 hours, about 14 hours, about 15 hours, about 16 hours, about 17 hours, about 18 hours, or about 24 hours. In an embodiment of the invention, the minimum course of treatment is about 1, about 2, about 3, about 4, about 5, about 6, or about 7 days, or about 1, about 2, about 3, about 4, about 5, about 6, about 7, or about 8 weeks, or about 1, about 2, about 3, about 4, about 5, about 6, about 7, about 8, about 9, about 10, about 11, about 12, about 18, or about 24 months.

In an embodiment of the invention, a nitric oxide treatment session is administered one or more times per day. In an embodiment of the invention, nitric oxide treatment session may be once, twice, three times, four times, five times, six times, or more than six times per day. In an embodiment of the invention, the treatment session may be administered once a month, once every two weeks, once a week, once every other day, daily, or multiple times in one day.

### Timing of a Pulse of NO

**In** an embodiment of the invention, the breath pattern is correlated with an algorithm to calculate the timing of administration of a dose of nitric oxide.

The precision of detection of an inhalation/inspiration event also permits the timing of a pulse of gas (e.g., NO) to maximize its efficacy by administering gas at a specified time frame of the total inspiration time of a single detected breath.

**In** an embodiment of the invention, at least fifty percent (50%) of the pulse dose of a gas is delivered over the first third of the total inspiratory time of each breath. In an embodiment of the invention, at least sixty percent (60%) of the pulse dose of a gas is delivered over the first third of the total inspiratory time. In an embodiment of the invention, at least seventy-five percent (75%) of the pulse dose of a gas is delivered over the first third of the total inspiratory time for each breath. In an embodiment of the invention, at least eighty-five (85%) percent of the pulse dose of a gas is delivered over the first third of the total inspiratory time for each breath. In an embodiment of the invention, at least ninety percent (90%) of the pulse dose of a gas is delivered over the first third of the total inspiratory time. In an embodiment of the invention, at least ninety-two percent (92%) of the pulse dose of a gas is delivered over the first third of the total inspiratory time. In an embodiment of the invention, at least ninety-five percent (95%) of the pulse dose of a gas is delivered over the first third of the total inspiratory time. In an embodiment of the invention, at least ninety-nine (99%) of the pulse dose of a gas is delivered over the first third of the total inspiratory time. In an embodiment of the invention, 90% to 100% of the pulse dose of a gas is delivered over the first third of the total inspiratory time.

In an embodiment of the invention, at least seventy percent (70%) of the pulse dose is delivered to the patient over the first half of the total inspiratory time. In yet another embodiment, at least seventy-five percent (75%) of the pulse dose is delivered to the patient over the first half of the total inspiratory time. In an embodiment of the invention, at least eighty percent (80%) of the pulse dose is delivered to the patient over the first half of the total inspiratory time. In an embodiment of the invention, at least 90 percent (90%) of the pulse dose is delivered to the patient over the first half of the total inspiratory time. In an embodiment of the invention, at least ninety-five percent (95%) of the pulse dose is delivered to the patient over the first half of the total inspiratory time. In an embodiment of the invention, 95% to 100% of the pulse dose of a gas is delivered over the first half of the total inspiratory time

In an embodiment of the invention, at least ninety percent (90%) of the pulse dose is delivered over the first two-thirds of the total inspiratory time. In an embodiment of the invention, at least ninety-five percent (95%) of the pulse dose is delivered over the first two-thirds of the total inspiratory time. In an embodiment of the invention, 95% to 100% of the pulse dose is delivered over the first two-thirds of the total inspiratory time.

When aggregated, administration of a number of pulse doses over a therapy session/timeframe can also meet the above ranges. For example, when aggregated greater than 95% of all the pulse doses administered during a therapy session were administered over the first two thirds of all of the inspiratory times of all of the detected breaths. In higher precision embodiments, when aggregated greater than 95% of all the pulse doses administered during a therapy session were administered over the first third of all of the inspiratory times of all of the detected breaths.

Given the high degree of precision of the detection methodologies of the present disclosure, a pulse dose can be administered during any specified time window of an inspiration. For example, a pulse dose can be administered targeting the first third, middle third or last third of a patient's inspiration. Alternatively, the first half or second half of an inspiration can be targeted for pulse dose administration. Further, the targets for administration may vary. In one embodiment, the first third of an inspiration time can be targeted for one or a series of inspirations, where the second third or second half may be targeted for one or a series of subsequent inspirations during the same or different therapy session. Alternatively, after the first quarter of an inspiration time has elapsed the pulse dose begins and continues for the middle half (next two quarters) and can be targeted such that the pulse dose ends at the beginning of the last quarter of inspiration time. In some embodiments, the pulse may be delayed by 50, 100, or 200 milliseconds (ms) or a range from about 50 to about 200 milliseconds.

The utilization of a pulsed dose during inhalation reduces the exposure of poorly ventilated areas of the lung and alveoli from exposure to a pulsed dose gas, e.g., NO. In one embodiment, less than 5% of poorly ventilated (a) areas of the lung or (b) alveoli are exposed to NO. In one embodiment, less than 10% of poorly ventilated (a) areas of the lung or (b) alveoli are exposed to NO. In one embodiment, less than 15% of poorly ventilated (a) areas of the lung or (b) alveoli are exposed to NO. In one embodiment, less than 20% of poorly ventilated (a) areas of the lung or (b) alveoli are exposed to NO. In one embodiment, less than 25% of poorly ventilated (a) areas of the lung or (b) alveoli are exposed to NO. In one embodiment, less than 30% of poorly ventilated (a) areas of the lung or (b) alveoli are exposed to NO. In one embodiment, less than 50% of poorly ventilated (a) areas of the lung or (b) alveoli are exposed to NO. In one embodiment, less than 60% of poorly ventilated (a) areas of the lung or (b) alveoli are exposed to NO. In one embodiment, less than 70% of poorly ventilated (a) areas of the lung or (b) alveoli are exposed to NO. In one embodiment, less than 80% of poorly ventilated (a) areas of the lung or (b) alveoli are exposed to NO. In one embodiment, less than 90% of poorly ventilated (a) areas of the lung or (b) alveoli are exposed to NO.

While preferred embodiments of the invention are shown and described herein, such embodiments are provided by way of example only and are not intended to otherwise limit the scope of the invention. Various alternatives to the described embodiments of the invention may be employed in practicing the invention.

The embodiments encompassed herein are now described with reference to the following examples. These examples are provided for the purpose of illustration only and the disclosure encompassed herein should in no way be construed as being limited to these examples, but rather should be construed to encompass any and all variations which become evident as a result of the teachings provided herein.

### EXAMPLES

### Example 1: Determination of Precise Breath Sensitivity for Appropriate Trigger/Arming Thresholds

A device using a threshold algorithm to detect breaths was used in this Example (Device 1). A threshold algorithm detects breaths using pressure; that is a pressure drop below a certain threshold must be met upon inspiration to detect and count a breath. That pressure threshold can be modified as a result of varying the detection sensitivity of the Device 1. Several breath sensitivity settings were tested in the present Example. Settings from 1 to 10 were tested, with 1 being the least sensitive and 10 being the most sensitive. The trigger threshold, shown in cm H₂O, is the threshold level at which nitric oxide is delivered. The arming threshold, also shown in cm H₂O, is the threshold level at which the device is armed for the next delivery of nitric oxide. The data are shown below in Table 1.

Table 1, below, illustrates a data set collected in this Example. Variation in the breath sensitivity setting resulted in an increase in trigger threshold (measured in cm H₂O) from -1.0 at the least sensitive setting (1) to -0.1 at the most sensitive setting (10). In addition, the arming threshold (measured in cm H₂O) stayed constant at 0.1 from a sensitivity setting of 1 through a setting of 6, and decreased by 0.02 for each sensitivity setting thereafter through 10. This indicates that the most sensitive breath sensitivity setting allows breaths to be detected more accurately, which leads to more accurate pulsatile delivery of nitric oxide in a shorter window of time, i.e., earlier in the inspiratory part of the breath. Based on these data, additional tests were performed at sensitivity settings of 8 and 10.

**Table 1: Breath Sensitivity and Trigger/Arming Thresholds**

| Breath Sensitivity | 1 | 2 | 3 | 4 | 5 | 6 | 7 | 8 | 9 | 10 |
|---|---|---|---|---|---|---|---|---|---|---|
| Trigger Threshold (cm H₂O) | -1.0 | -0.9 | -0.8 | -0.7 | -0.6 | -0.5 | -0.4 | -0.3 | -0.2 | -0.1 |
| Arming Threshold (cm H₂O) | +0.1 | +0.1 | +0.1 | +0.1 | +0.1 | +0.1 | +0.08 | +0.06 | +0.04 | +0.02 |

The conclusion is that a higher breath sensitivity setting correlates to a lower trigger threshold and a higher arming threshold, which prepares the device to deliver short, precise pulses of nitric oxide over the therapy treatment course.

### Example 2: Testing a Device Against Various Breath Patterns

As discussed above, accurate and timely delivery of nitric oxide is critical to the present invention. In order to ensure that a device will deliver a precise dose of gas within a precise window of time, ten different breath patterns were tested using a mechanical lung and nose model. Ten different simulated breath patterns were analyzed, and the breath patterns had varying respiratory rate (8 to 36 bpm), tidal volume (316 to 912 ml), and Inspiration: Expiration (I/E) ratios (1:1 to 1:4). These variable breath patterns are patterns expected for subjects age 16 and up and are summarized in Table 2. Real world conditions were emulated to the extent possible.

**Table 2: Summary of Breath Patterns Tested**

| **Respiratory Rate (bpm)** | **Male/ Female** | **Height (cm)** | **Ideal Body Weight (kg)** | **Tidal Volume (mL)** | **Inspiration Time (sec)** | **I:E Ratio** |
|---|---|---|---|---|---|---|
| 8 | F | 174 | 68.1 | 456 | 1.5 | 1:4 |
| 8 | M | 186 | 86.4 | 564 | 1.5 | 1:4 |
| 12 | F | 152 | 51.9 | 316 | 1.25 | 1:3 |
| 12 | M | 186 | 86.4 | 564 | 1.25 | 1:3 |
| 18 | F | 174 | 68.1 | 456 | 1.1 | 1:2 |
| 18 | M | 186 | 86.4 | 564 | 1.1 | 1:2 |
| 24 | F | 152 | 51.9 | 316 | 1.0 | 1:1.5 |
| 24 | F | 174 | 68.1 | 456 | 1.0 | 1:1.5 |
| 36 | F | 152 | 51.9 | 632 | 0.8 | 1:1 |
| 36 | F | 174 | 68.1 | 912 | 0.8 | 1:1 |

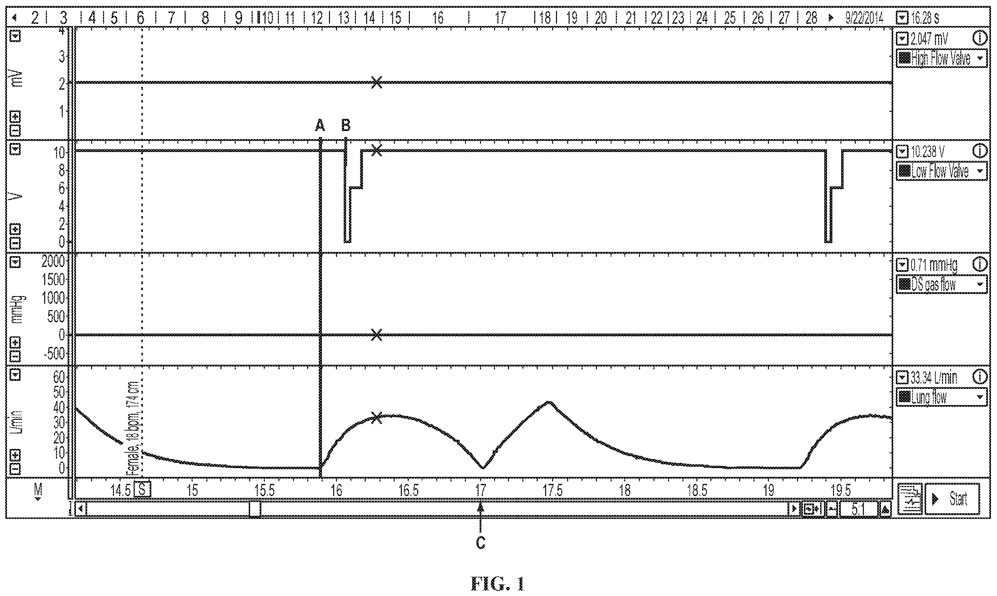

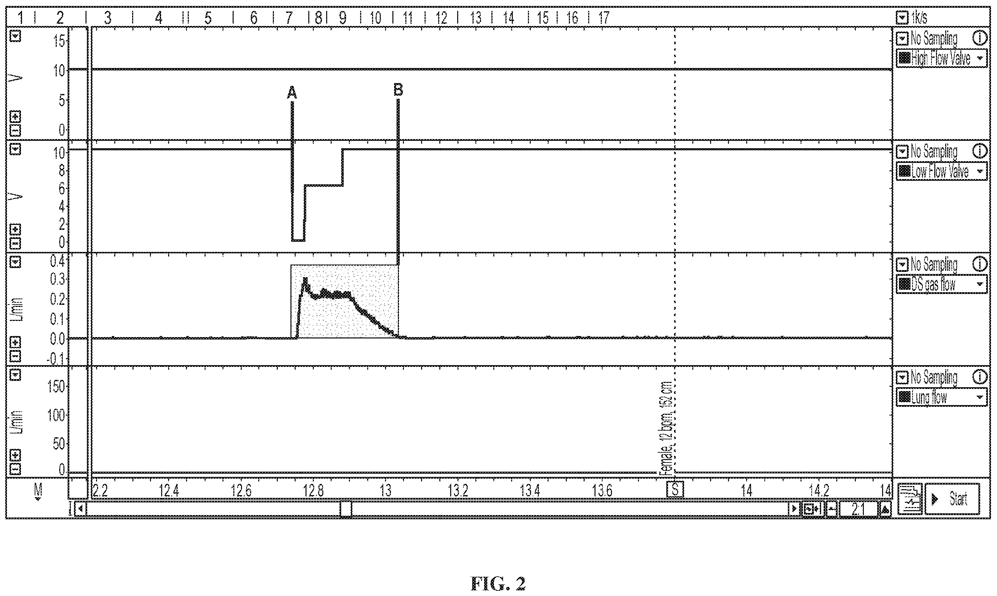

Two devices were tested - Device 1 was tested at sensitivity level 8 and sensitivity level 10, and the other device (Device 2, which further includes a slope algorithm) was tested at sensitivity level 10. The investigation consisted of two parts. Part 1 measured the time delay between the initiation of the inspiratory breath and the onset of nitric oxide delivery using the 10 different simulated respiratory patterns. This time delay is measured using two data points - the time between initiation of inspiration (FIG. 1, Point A) and breath detection with concurrent opening of the delivery valve (FIG. 1, Point B). Part 2 measured the duration and volume of the delivered pulse covering the same breath patterns in Table 2. The time duration of the gas pulse is measured, from breath detection and concurrent opening of the delivery valve, which corresponds to the initiation of gas delivery, (FIG. 2, Point A) to the completion of the gas delivery (FIG. 2, Point B). The volume of the delivered pulse is measured by integration of the gas flow over the pulse duration. In addition, data from Part 1, measured time delay, and Part 2, measured pulse duration, are added to calculate the dose delivery time, sometimes referred to as "delivered pulse width".

Part 1: Measuring time delay between initiation of inspiration and onset of NO delivery. This portion of the test was conducted at a dose of 75 µg/kg-IBW/hr with a drug concentration input of 6 mg/L (4880 ppm). This test was conducted using nitrogen only. The primary output for Part 1 is time duration between initiation of inspiration and valve opening/breath detection indication. Point A in FIG. 1 is the point where the lung air flow rises just above resting line. The time of valve opening is indicated as Point B in FIG. 1 and is displayed as a sudden voltage drop in the detector. The time interval between Point A and Point B is the valve time delay, or trigger delay, and is calculated for each breath pattern. The total inspiratory time corresponds to the interval from Point A to Point C (which is the end of inspiration).

Part 2: Measuring the duration and volume of the delivered pulse. The same breath patterns were used in this part of the investigation. Doses of 10, 15, 30 and 75 µg/kg-IBW/hr were tested. The device was programmed for each dose, patient IBW, and respiratory rate (breaths per minute). The resulting pulsatile gas flow was determined by a flow meter. The pulse duration is the time between the point at which the valve opening was indicated, displayed as a sudden voltage drop in the detector, corresponding to Point A in FIG. 2, and the time at which the gas flow returns to baseline at Point B in FIG. 2. The volume of the delivered pulse is the integrated gas flow during the pulse duration. The pulse duration was added to the pulse delay from Part 1 to give the dose delivery time or "delivered pulse width." FIG 1. illustrates the results of Part 1. There are four panels shown in FIG. 1. The second and fourth panels show the breath detection which corresponds to the flow control valve operation and a representation of a breath pattern, respectively. Point A shows initiation of inspiration, Point B shows breath detection which corresponds to the opening of the flow valve, and Point C shows the end of inspiration. From this data, the time delay between points A and B can be calculated.

FIG 2. illustrates the results of Part 2. There are four panels shown in FIG. 2. The second and third panels show the breath detection which corresponds to the flow control valve operation and a representation of the pulsatile gas flow, respectively. Point A shows breath detection which corresponds to the opening of the flow valve and Point B shows the end of pulsatile flow. From this data, the pulse duration between points A and B can be calculated.

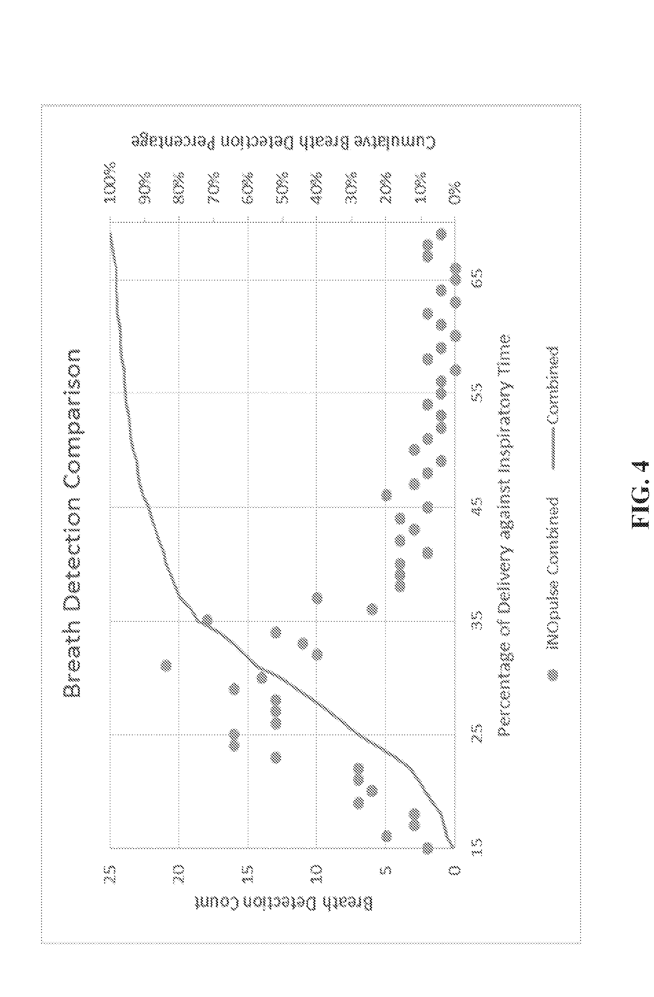

**Table 3, below, summarizes the results depicted in FIG. 3 and FIG. 4.**

| | **% Delivery of NO Within Portion of Inspiratory Time** | | |
|---|---|---|---|
| **Device** | **First Third** | **First Half** | **First Two-thirds** |
| Device 1 (Sensitivity 8) | 17 | 77 | 95 |
| Device 1(Sensitivity 10) | 62 | 98 | 100 |
| Device 2 | 93 | 100 | 100 |
| Combined Data | 64 | 93 | 99 |

FIG. 3 depicts results for the breath detection count for each device listed in Table 3. The Device 2, or the green data in FIG. 3, illustrates that at least 93% of nitric oxide is delivered within the first third of the inspiratory portion of the breath. 100% of the nitric oxide is delivered within the first half of the inspiratory portion of the breath. Comparatively, for the Device 1 at a sensitivity setting of 8, at least 17% of the nitric oxide is delivered within the first third of the inspiratory portion of the breath, at least 77% within the first half, and at least 95% within the first two-thirds of the inspiratory portion of the breath. The Device 1 at a sensitivity setting of 10 showed results that at least 62% of nitric oxide is delivered within the first third of the inspiratory portion of the breath, at least 98% in the first half, and 100% in the first two-thirds of the inspiratory portion of the breath. FIG. 4 depicts the combined data curve for all three tests.

This data concludes that lower doses of nitric oxide are needed over the course of a single treatment because more nitric oxide is being more precisely delivered with each pulse over a shorter period of time during the course of treatment. Lower doses of nitric oxide may lead to use of less drug overall, and also may lead to less risk of detrimental side effects.

### Example 3: Improvement to Oxygen Saturation in Patients With Severe Hypoxemia

Patients suffering from pulmonary hypertension associated with idiopathic pulmonary fibrosis (PH-IPF), and patients diagnosed pulmonary arterial hypertension (PAH) as WHO Diagnostic Group 1 were tested in this study example. The patients included in this study were all on long term oxygen therapy (LTOT) and have been on LTOT for at least 3 months and for at least 10 hours per day. The results of this study, and the SpO₂ measurements specifically, were derived from a clinical study measuring the effects of iNO on functional respiratory imaging parameters in certain patient populations.

PH-IPF patients were tested according to the following procedure per protocol Pulses-COPD-006 Part 2: A baseline measurement of SpO₂ was taken within 24 hours of the start of the study. A 6-minute walk test (6MWT) was given, and SpO₂ measurements were taken every minute during the 6MWT. Following baseline measurements, the subjects were put on either 30 mcg/kgIBW/hr or 75 mcg/kgIBW/hr of iNO for 4 weeks, along with their LTOT they were already receiving. 6MWT and SpO₂ measurements were taken again at 2 weeks and 4 weeks, and iNO was then discontinued. The 6MWT and SpO₂ measurements were taken again at 6 weeks after 2 weeks of only the LTOT.

The PAH patients were tested according to the following procedure per protocol Pulse-PAH-201: A baseline measurement of SpO₂ was taken within 24 hours of the start of the study. A 6-minute walk test (6MWT) was given, and SpO₂ measurements were taken at baseline and at the end of the 6MWT. Following baseline measurements, the subjects were put on either 25 mcg/kgIBW/hr or 75 mcg/kgIBW/hr of iNO for 16 weeks, along with their LTOT they were already receiving. 6MWT and SpO₂ measurements were taken at 4 weeks, 8 weeks, 12 weeks, and 16 weeks.

Results indicated that drops in oxygen saturation during the 6MWT were blunted by use of the pulsed dose iNO. Table 4, below, shows the change in SpO₂ Nadir during the 6MWT for PAH subjects on placebo, iNO25 (25mcg/kgIBW/hr), and iNO75 (75mcg/kgIBW/hr). All test subjects showed improvement after 16 weeks of treatment with half showing improvement of 5% or more in the iNO75 cohort.

**Table 4**

| | Drop or no change in SpO₂ Nadir | Improvement in SpO₂ Nadir | Improvement in SpO₂ Nadir (2% or more) | Improvement in SpO₂ Nadir (5% or more) |
|---|---|---|---|---|
| Placebo | 66% | 34% | 22% | 10% |
| iNO 25 | 40% | 60% | 50% | 10% |
| iNO 75 | 0% | 100% | 88% | 50% |

Table 5, below, shows the improvement in oxygen saturation during the 6MWT for 2 PH-IPF patients, one on iNO75 and one on iNO30 (30mcg/kgIBW/hr). The results show both subjects saw improvement in the SpO₂ Nadir on iNO compared to baseline with an average improvement of 5.5%. In addition, the level of oxygen desaturation during exercise improved for both subjects, representing an average improvement of 28.5%.

**Table 5**

| | **iNO 75** | **iNO 30** |
|---|---|---|
| **Average hours of use per day** | 23.8 hrs/day | 12.8 hrs/day |

| **6MWD (meters)** | | |
|---|---|---|
| **Baseline** | 98 m | 510 m |
| **4 Weeks** | 197 m | 560 m |
| **Meters improvement at 4 weeks** | **99 m** | **50 m** |

| **Nadir SpO2 (% SpO₂)** | | |
|---|---|---|
| **Baseline** | 76% | 83% |
| **4 Weeks** | 79% | 89% |
| **Percent Improvement at 4 weeks** | **4%** | **7%** |

| **Oxygen desaturation during 6MWT (% SpO₂)** | | |
|---|---|---|
| **Baseline** | 23% | 15% |
| **4 Weeks** | 16% | 11% |
| **Percent Improvement at 4 weeks** | **30%** | **27%** |

Table 6, below, shows results for distance saturation product (DSP) for 2 subjects with PH-IPF (the same subjects as Table 5). The DSP is calculated by multiplying the distance (6MWD) by the nadir in the oxygen saturation during the 6MWT. DSP has been shown to be a better predictor of long term outcomes than 6MWD alone. Both subjects showed an average DSP improvement of 78.1m% with iNO. DSP is a composite measurement that shows iNO improves oxygen saturation in conjunction with exercise capacity.

**Table 6**

| | **iNO 75** | **iNO 30** |
|---|---|---|
| Average hours of use per day | 23.8 hrs/day | 12.8 hrs/day |

| **Distance Saturation Product [DSP] (meter%)** | | |
|---|---|---|
| Baseline | 74.5 m% | 423.3 m% |
| 4 Weeks | 155.6 m% | 498.4 m% |
| Improvement at 4 weeks (meter%) | 81.1 m% | 75.1 m% |

The data indicate that pulsed delivery of iNO in an outpatient setting significantly improves oxygen saturation in patients suffering from hypoxemia who show little or no improvement on long-term oxygen therapy alone. The data also show that DSP is vastly improved by an average of 78.1m% over a baseline measurement with LTOT alone within a 4-week period.

## Claims

1. Inhaled nitric oxide (iNO) for use in a method of improving oxygen saturation in a patient having an initial oxygen saturation of less than 88% when receiving continuous oxygen therapy at a flow of 10L/min., said method comprising:
(a) administering said iNO to said patient until the oxygen saturation is at least 88%, wherein said iNO is delivered in a continuous manner or in a pulsatile manner; and
(b) continuing said oxygen administration together with iNO administration, wherein said iNO is delivered in a pulsatile manner during a portion of the inspiratory phase of a breath, wherein:
(i) delivery of at least fifty percent of the dose of iNO occurs within the first third of the total inspiratory phase of a breath;
(ii) delivery of the dose of iNO occurs within the first two-thirds of the total inspiratory phase of a breath;
(iii) delivery of at least ninety percent of the dose of iNO occurs within the first two-thirds of the total inspiratory phase of a breath; or
(iv) delivery of at least seventy percent of the dose of iNO occurs within the first half of the total inspiratory phase of a breath.

2. The iNO for use according to claim 1, wherein in step (b), delivery of the dose of iNO occurs within the first third of the inspiratory phase of a breath.

3. The iNO for use according to claim 1, wherein in step (b), the iNO is delivered in a series of pulses.

4. The iNO for use according to claim 1, wherein in step (b), the iNO is delivered at 75 mcg/kg/hr or 25 mcg/kg/hr.

5. The iNO for use according to claim 1, wherein the patient has idiopathic pulmonary fibrosis (IPF), pulmonary arterial hypertension (PAH), pulmonary hypertension (PH), chronic obstructive pulmonary disorder (COPD), cystic fibrosis (CF), interstitial lung disease (ILD), combined pulmonary fibrosis and emphysema (CPFE), chronic high altitude sickness, chronic thromboembolic pulmonary hypertension (CTEPH), emphysema, or pneumonia.

## Patentansprüche

1. Inhalatives Stickstoffmonoxid (iNO) zur Verwendung in einem Verfahren zur Verbesserung der Sauerstoffsättigung bei einem Patienten mit einer anfänglichen Sauerstoffsättigung von weniger als 88 % unter kontinuierlicher Sauerstofftherapie mit einem Fluss von 10L/min, das Verfahren umfassend:
(a) Verabreichen des iNO an den Patienten, bis die Sauerstoffsättigung mindestens 88% ist, wobei das iNO kontinuierlich oder pulsierend abgegeben wird; und
(b) Fortsetzen der Sauerstoffverabreichung zusammen mit der iNO-Verabreichung, wobei das iNO pulsierend während eines Teils der Inspirationsphase eines Atemzugs abgegeben wird, wobei:
(i) Abgabe von mindestens fünfzig Prozent der iNO-Dosis innerhalb des ersten Drittels der gesamten Inspirationsphase eines Atemzugs erfolgt;
(ii) Abgabe der iNO-Dosis innerhalb der ersten zwei Drittel der gesamten Inspirationsphase eines Atemzugs erfolgt;
(iii) Abgabe von mindestens neunzig Prozent der iNO-Dosis innerhalb der ersten zwei Drittel der gesamten Inspirationsphase eines Atemzugs erfolgt; oder
(iv) Abgabe von mindestens siebzig Prozent der iNO-Dosis innerhalb der ersten Hälfte der gesamten Inspirationsphase eines Atemzugs erfolgt.

2. iNO zur Verwendung nach Anspruch 1, wobei in Schritt (b) Abgabe der iNO-Dosis innerhalb des ersten Drittels der Inspirationsphase eines Atemzugs erfolgt.

3. iNO zur Verwendung nach Anspruch 1, wobei in Schritt (b) das iNO in einer Reihe von Impulsen abgegeben wird.

4. iNO zur Verwendung nach Anspruch 1, wobei in Schritt (b) das iNO bei 75 mcg/kg/h oder 25 mcg/kg/h abgegeben wird.

5. iNO zur Verwendung nach Anspruch 1, wobei der Patient an idiopathischer Lungenfibrose (IPF), pulmonaler arterieller Hypertonie (PAH), pulmonaler Hypertonie (PH), chronisch obstruktiver Lungenerkrankung (COPD), Mukoviszidose (CF), interstitieller Lungenerkrankung (ILD), kombinierter Lungenfibrose und Emphysem (CPFE), chronischer Höhenkrankheit, chronischer thromboembolischer pulmonaler Hypertonie (CTEPH), Emphysem oder Lungenentzündung leidet.

## Revendications

1. Oxyde nitrique inhalé (iNO) à utiliser dans un procédé visant à améliorer la saturation en oxygène chez un patient présentant une saturation initiale en oxygène inférieure à 88 % lors d'une oxygénothérapie continue à un débit de 10L/min., ledit procédé comprenant :
(a) l'administration dudit iNO audit patient jusqu'à ce que la saturation en oxygène soit d'au moins 88 %, dans lequel ledit iNO est délivré de manière continue ou pulsatile ; et
(b) la poursuite de l'administration d'oxygène conjointement à l'administration d'iNO, dans lequel ledit iNO est délivré de manière pulsatile pendant une partie de la phase inspiratoire d'une respiration, dans lequel :
(i) l'administration d'au moins cinquante pour cent de la dose d'iNO se produit dans le premier tiers de la phase inspiratoire totale d'une respiration ;
(ii) l'administration de la dose d'iNO a lieu dans les deux premiers tiers de la phase inspiratoire totale d'une respiration ;
(iii) l'administration d'au moins quatre-vingt-dix pour cent de la dose d'iNO a lieu dans les deux premiers tiers de la phase inspiratoire totale d'une respiration ; ou
(iv) l'administration d'au moins soixante-dix pour cent de la dose d'iNO se produit dans la première moitié de la phase inspiratoire totale d'une respiration.

2. iNO à utiliser selon la revendication 1, dans lequel, à l'étape (b), l'administration de la dose d'iNO a lieu dans le premier tiers de la phase inspiratoire d'une respiration.

3. iNO à utiliser selon la revendication 1, dans lequel, à l'étape (b), l'iNO est délivré en une série d'impulsions.

4. iNO à utiliser selon la revendication 1, dans lequel, à l'étape (b), l'iNO est délivré à 75 mcg/kg/hr ou 25 mcg/kg/hr.

5. iNO à utiliser selon la revendication 1, dans lequel le patient souffre de fibrose pulmonaire idiopathique (FPI), d'hypertension artérielle pulmonaire (HTAP), d'hypertension pulmonaire (HTP), de bronchopneumopathie chronique obstructive (BPCO), de fibrose kystique (FK), de pneumopathie interstitielle diffuse (PID), de fibrose pulmonaire et d'emphysème combinés (FPEC), de mal chronique de haute altitude, d'hypertension pulmonaire thromboembolique chronique (HTPTEC), d'emphysème ou de pneumonie.
